# EUROPEAN PATENT APPLICATION

(11) **EP 3 292 902 A1**
(43) Date of publication of application: **14.03.2018**
(21) Application number: 16187812.9
(22) Date of filing: 08.09.2016
(51) Int. Cl.: B01D 53/14, C10G 53/12, C10G 75/04

(54) **METHOD FOR REDUCING AND/OR INHIBITING ALDOL CONDENSATION IN GAS SCRUBBERS**

(71) Applicant: Borealis AG, 1220 Wien (AT)
(72) Inventor: REGUILLO CARMONA, Rebeca, 4020 Linz (AT); DICKE, René, 4060 Leonding (AT)
(74) Representative: Maikowski & Ninnemann Patentanwälte Partnerschaft mbB

(57) **Abstract**

The present invention relates to the use of at least one ketone as inhibitor for aldol condensation of at least one aldehyde present in a gas scrubbing solution, in particular an acid gas scrubbing solution.

## Description

The present invention relates to the use of at least one ketone as inhibitor of aldol condensation according to claim 1 and a method for reducing and/or inhibiting aldol condensation according to claim 10.

### Description

The occurrence of so-called red oil polymers is common for caustic towers in steam cracking units. The primary cause for red oil formation are aldehydes and conjugated vinyl monomers such acetaldehyde and vinyl acetate present in the crack gas. Aldehydes such as acetaldehyde present in the gas feed react with the caustic in the solution via aldol condensation reaction giving rise to the so-called red oil polymer. The chemical mechanism of this reaction is shown below:

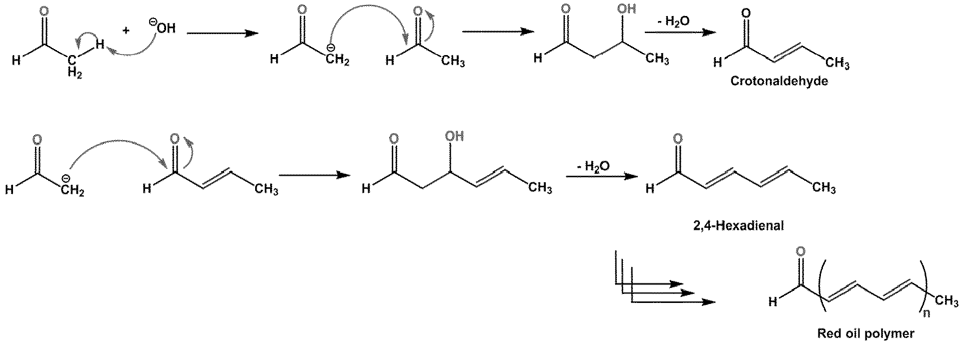

The progression of the condensation reaction yields long chain and high molecular weight polymeric molecules. These compounds are called red oils. They have a highly undefined structure and have a yellowish to brownish/red color depending on their molecular weight and double bond system. As the reaction progresses, the color of the solution will become darker until an oily, viscous hydrocarbon liquid phase is produced. If left unchecked, an aldehyde resin material is formed. This is the fouling material that composes deposits found in many acid gas scrubber systems, especially the so called caustic towers.

The majority of the red oil formation occurs in the lower section of the caustic tower and can adversely impact the caustic tower operation mostly through deposition of polymers and mechanical blocking - e.g. in the down corner areas and on the trays.

The problems associated with red oil formation in acid gas scrubbers or caustic towers are normally addressed by adding commercial anti-red oil additives. Anti-red oil additives are chemical substances that react with the red oil precursors inhibiting the polymerization reaction. This chemical treatment consists usually but is not limited to an amine or amine derivate. It reacts with the active aldehyde group to form an inert product, generally an oxime. The product (oxime) formed is soluble in water and in caustic solutions, thus, the spent caustic blowdown removes the oxime product from the tower. The chemical reaction between the carbonyl group of acetaldehyde and a typical amine-type carbonyl scavenger is shown below:

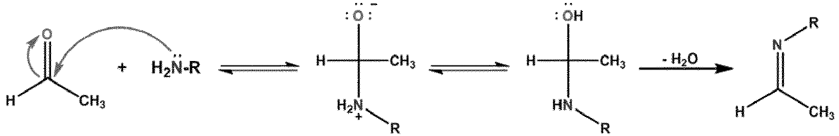

This chemical reaction is stoichiometric and requires 1:1 equivalents of scavenger per acetaldehyde. The predominant anti-red oil additives are usually generic primary, secondary or tertiary amines, although ammonium salts can be also used for this purpose. Commercially available anti-red oil additives are listed below in Table 1.

**Table 1: Typical anti-red oil products**

| | **Formulas** | **Structures** |
|---|---|---|
| **Amines** | | |
| **Primary amines** | | |
| Monoethanolamine (MEA) | H₂NCH₂CH₂OH | |
| Diglycolamine (DGA) | H₂NCH₂CH₂OCH₂CH₂OH | |
| 2-amino-2-methyl-1-propanol (AMP) | HOCH₂C[CH₃]₂NH₂ | |

| **Secondary amines** | | |
|---|---|---|
| Diethanolamine (DEA) | HN[CH₂CH₂OH]₂ | |
| Diisopropanolamine (DIPA) | [CH₃CH(OH)CH₂]₂NH | 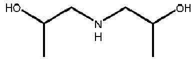 |
| Methylethanolamine | HNCH₃CH₂CH₂OH | 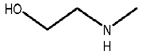 |

| **Tertiary Amines** | | |
|---|---|---|
| Methyldiethanolamine (MDEA) | CH₃N[CH₂CH₂OH]₂ | 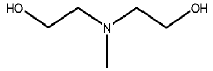 |
| Triethanolamine (TEA) | N[CH₂CH₂OH]₃ | 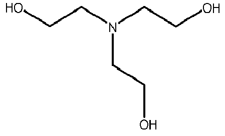 |

| **Ammonium salts** | | |
|---|---|---|
| Hydroxyl amine sulphate | (H₃NOH)₂SO₄ | |

The use of other kind of additives has also been reported, like Si-based products (EP1102063 A1, US 5,527,447).

The major problems associated with these kind of additives is the interaction of the remaining commercial products with downstream units in the ethylene plant. Although these additives are typically soluble in water, it may occur that when hydrocarbon-water phase separation is not efficient (due to emulsion formations, presence of rag layers, etc) or additive overdose happens (feeding system failure, miscalculation of additive amount, etc), part of these products end up in downstream units where they can cause damage or malfunction (e.g. deactivation of hydrogenation catalysts, additive carried over in the pygas stream recirculated to the primary fractionator, etc.).

Furthermore, the so-formed red oil polymer accumulates in the bottom part of the caustic towers and needs to be removed on a regular basis. A too high amount of formed solid aldol polymer can lead to operational issues for instance due to viscosity increase or mixing issues. Therefore, the operators have to invest additional time for pumping the polymer layer into the downstream vessels.

Thus, there is a need for alternative methods for reducing and inhibiting red oil formation in a gas scrubber, in particular in an acid gas scrubber.

The object of the present invention is to provide an alternative method for reducing and/or inhibiting red oil formation in a gas scrubber.

This object is being solved by the use of at least one ketone according to claim 1 and a method for inhibiting and/or reducing aldol condensation in a gas scrubber according to claim 8.

Accordingly, at least one ketone is used as an inhibitor for aldol condensation of at least one aldehyde present in a gas scrubbing solution, in particular an acid gas scrubbing solution in a gas scrubber.

The at least one ketone applied as inhibitor for aldol condensation comprises preferably the structural unit -CO-CH-. Thus, the ketone used as red oil inhibitor comprises at least one H-atom on the α-carbon atom to the carbonyl group.

In an embodiment the at least one ketone is selected from a group of compounds of the general formulae (I)

R¹-CO-CHR²R³

wherein the moiety R¹ is selected from a group comprising linear or branched C₁-C₁₀ alkyl, C₃-C₁₀-cycloalkyl and linear or branched C₁-C₁₀-alkyl substituted C₃-C₁₀-cycloalkyl,
wherein the moieties R² and R³ are selected from a group comprising H, linear or branched C₁-C₁₀ alkyl, C₃-C₁₀-cycloalkyl and linear or branched C₁-C₁₀-alkyl substituted C₃-C₁₀-cycloalkyl, and
wherein R¹, R² and R³ can be the same or different, or
wherein R¹ and R² form together a C₄-C₁₀ cycloalkyl ring.

The linear or branched C₁-C₁₀-alkyl, C₃-C₁₀-cycloalkyl, C₁-C₁₀-alkylsubstituted C₃-C₁₀-aryl may be in each case interrupted by one or multiple oxygen atoms, sulphur atoms and/or substituted nitrogen atoms and/or by one or multiple groups of the type -C(O)O-, -OC(O)-, -C(O)- and/or -OC(O)O- and/or can be functionalized by one or multiple hydroxyl groups, amino groups, in particular primary amino groups or secondary amino groups, mercapto groups and/or halids, in particular Cl or Br.

In a further embodiment the moieties R¹, R² and R³ are selected from the group comprising H (in case of R² and R³), linear or branched C₁-C₅ alkyl and C₃-C₇-cycloalkyl. The moieties R¹, R² and R³ are in particular selected from a group comprising H (in case of R² and R³), ethyl, propyl, isopropyl, butyl or *iso*-butyl.

In a yet further embodiment the moieties R¹, R² and R³ are selected from the group of linear or branched C₁-C₅ alkyl and C₃-C₇-cycloalkyl substituted with at least one hydroxy group or at least one amino group, in particular at least one primary amino group or secondary amino group, such as hydroxy-C1-C3, (monoethylamino)-C1-C3,(diethylamino)-C1-C3.

In another embodiment the moieties R¹ and R² or R¹ and R³ are part of a C₅-C₈ cyclic ring system. Thus, in this case a cyclic ketone is used as inhibitor.

In a most preferred embodiment the at least one ketone is acetone, butan-2-one, pentan-2-one, pentan-3-one, hexan-2-one, hexan-3-one, 2-methyl-pentan-3-one, 2,2-dimethylpentan-3-one, 3-methyl-butanone, 3,3-dimethyl-butanone, cyclopentanone, cyclohexanone, 2-methyl-cyclohexanone, hydroxyacetone, 4-hydroxy-butan-2-one, (diethylamino)-acetone, (monoethylamino)-acetone, (monomethyl)-acetone.

In a further embodiment the at least one aldehyde causing the red oil formation is at least one low molecular weight aldehyde, in particular acetaldehyde, propanaldehyde, butanaldehyde, or a higher molecular weight aldehydes, preferably hexanal, heptanal, octanal nonanal, decanal, and/or C₁₅-C₂₀ aldehydes.

Esters compounds, in particular unsaturated ester compounds, may also cause or may be involved in red oil formation. Typical esters are for example compounds comprising at least one double bound that is preferably in conjugation to the -CO- group. Such ester may be arise from the reaction of an olefin and the corresponding acid, such as ethylene and acetic acid. A preferred ester is a conjugated vinyl monomer, such as vinyl acetate.

The aldol condensation reaction, which leads to the red oil formation, takes place by selfcondensation of aldehydes, ketones and/or unsaturated esters In order to initiate the aldol condensation reaction in basic media the presence of a hydrogen atom in α-position to the carbonyl group is required:

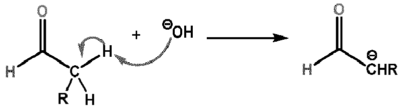

The first step of the aldol condensation reaction takes place by condensation through the methylene group in α- position to the carbonyl group of one aldehyde with the aldol group of another aldehyde molecule:

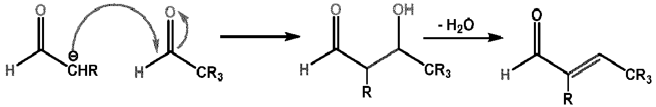

For the red oil polymer to be formed, the aldol condensation proceeds by reaction of the remaining aldehyde with the aldol groups of the intermediate products:

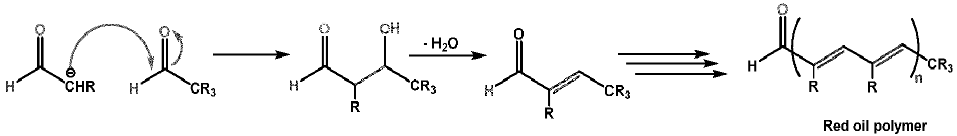

When a ketone, such as acetone is present, the aldol condensation reaction cannot proceed further after the first reaction step due to the fact that the resultant intermediate product is a ketone and not an aldehyde:

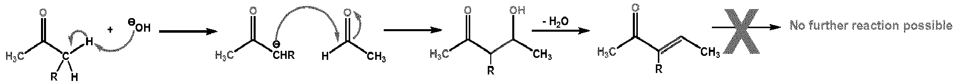

In this way, by the presence of a ketone, such as acetone in the reaction mixture, a major part of the aldehyde, such as acetaldehyde molecules will be compromised in the reaction with acetone, and will not be available for further reaction to produce long-chain red oil polymer. This will be translated in a low to none formation of red oil polymer in the reaction media.

As mentioned above, the use of acetone as inhibiting agent is particularly preferred since acetone is highly soluble in water. Thus, a carry over with the hydrocarbon phase is very unlikely. In addition to that, it has less impact in downstream units than other additives (e.g. less to no harm to catalysts or primary fractionator). Furthermore, acetone can be used as anti-red oil additive and depending on the fed amount of acetone the chain length of the aldol polymer can be regulated so that probably the foaming tendency is influenced positively.

Additionally, by controlling the chain length of the red oil polymer the viscosity of the processed solution can be controlled, so that an operator can manage the process more efficiently.

The object of the invention is also solved by a method for reducing and/or inhibiting aldol condensation of aldehydes, in particular low molecular weight aldehydes, in a gas scrubber solution, in particular an acid gas scrubber solution (caustic towers), wherein at least one ketone is fed to the gas scrubbing solution, in particular to the acid gas scrubbing solution.

The at least one ketone used in the present method is a ketone as defined previously.

In a further embodiment the at least one ketone as inhibitor for aldol condensation can be used as mixture with other process additives like anti-red-oil agents, anti-oxidants, or antifoaming agents.

In an embodiment of the present method the at least one ketone is fed to the gas scrubbing solution in a molar amount that is at least substantial the same as the molar amount of the at least one aldehyde in the gas scrubbing solution. Thus, the amount of the at least one ketone added to the gas scrubber solution should be sufficient to inhibit the aldol condensation of the at least one aldehyde.

In a variant the at least one ketone is fed to the gas scrubbing solution in a basic media. In particular, the at least one ketone is fed to the gas scrubbing solution in a NaOH containing solution.

In a further embodiment of the present method the at least one ketone is fed at different feeding points to the gas scrubber and/or gas scrubber solution. It is possible that the at least ketone as red oil inhibitor can be injected in the caustic tower through different feeding lines at multiple different points.

In a first variant the at least one ketone is fed into the fresh caustic stream that is subsequently fed to the caustic tower.

In a second variant the at least one ketone is fed into one or more of the recirculating caustic lines of the different sections in the caustic tower.

There may be one feeding point for the at least one ketone at one line or multiple feeding points at multiple lines transporting the recirculating caustic from different sections of the caustic tower. For example, there may be at least one feeding point for the at least one ketone at the line for recirculation the caustic compound from and into the lower section of the caustic tower. There also may be at least one feeding point for the at least one ketone at the line for recirculation the caustic compound from and into the middle and/or upper section of the caustic tower.

In yet a third further variant the at least one ketone is fed into the feed line of the hydrocarbon product entering the caustic tower.

In general a combination of two or all feeding variants is also possible and conceivable.

The present invention is explained in more detail in the following examples with reference to the figures. It shows:
- Figure 1: a process scheme of a scrubber unit; and

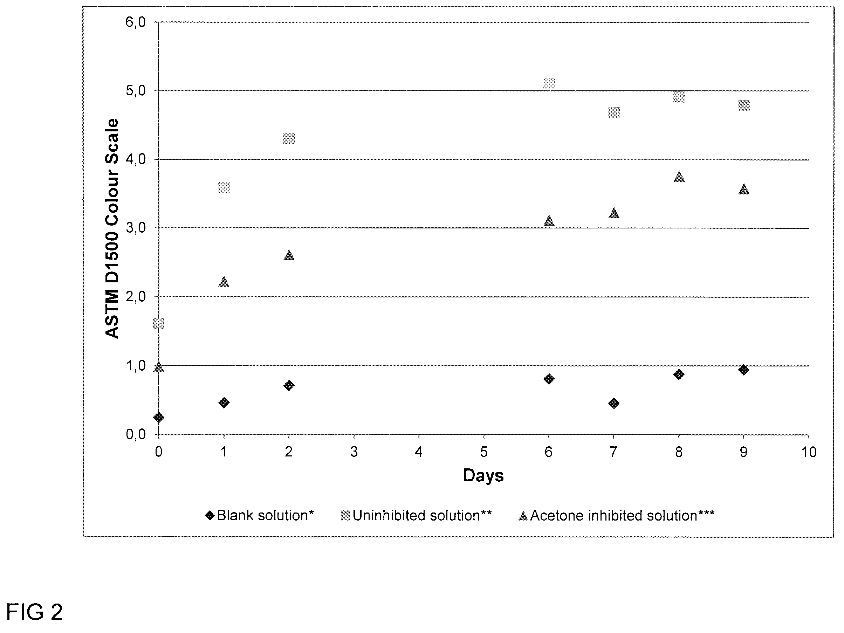

- Figure 2: a diagram showing comparative experimental results.

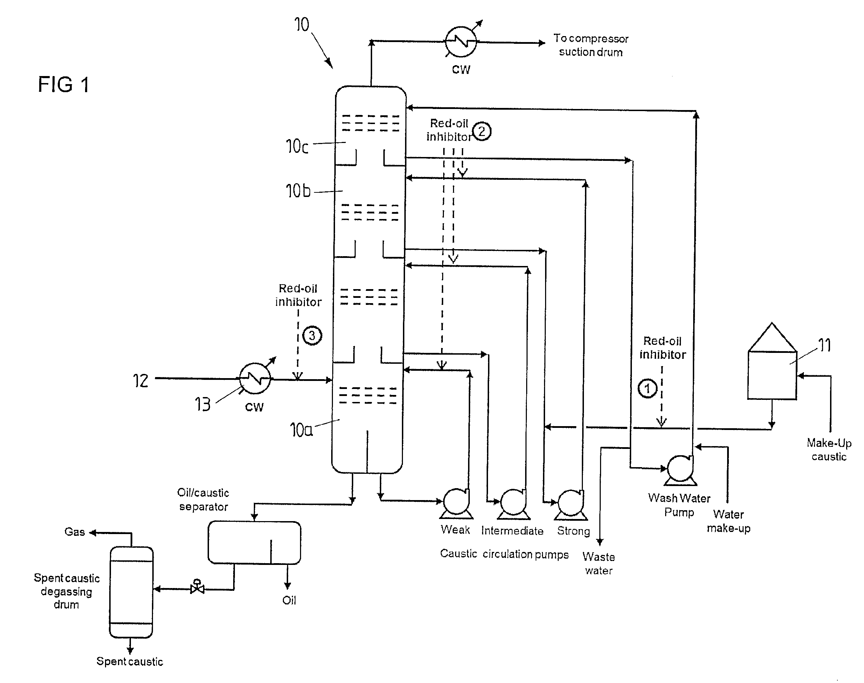

In the general scheme of Figure 1 a scrubber unit with a caustic tower 10 is illustrated. The scheme depicts the gas scrubber (caustic tower) as part of the refinery section.

In an embodiment of the present method the at least ketone as red oil inhibitor can be injected in the caustic tower through a feeding line at multiple different points 1, 2, 3.

The first feeding point 1 for the red oil formation inhibitor, such as acetone, is provided at a suitable location along the feeding line for the fresh caustic stream fed from the fresh caustic storage 11 to the caustic tower. Here the inhibitor is directly fed into the fresh caustic stream that is subsequently fed to the caustic tower 10.

The second feeding point 2 for the red oil inhibitor may be provided at any suitable location of one or more recirculating caustic lines. There may one feeding point at one or multiple feeding points at multiple lines transporting the recirculating caustic from different sections of the caustic tower.

One second feeding point 2a for the inhibitor is provided at the line for recirculation the caustic compound from and into the lower section 10a of the caustic tower 10. Further second feeding points 2b, c for the inhibitor may be located at the line for recirculation the caustic compound from and into the middle and/or upper section of the caustic tower 10 b,c.

The third feeding point 3 for the inhibitor is provided at the line for the hydrocarbon feed (such as ethylene) into the caustic tower 10. In particular, the inhibitor is fed into the hydrocarbon feed coming from the compressor 12 and preheater 13, i.e. after compressor 12 and preheater 13.

A combination of the different feeding points 1-3 is also possible.

### Example:

### a) Test method

The test method ASTM D1500 covers the visual determination of the colour of a wide variety of petroleum products, such as lubricating oils, heating oils, diesel fuel oils, and petroleum waxes. Using a standard light source, a liquid sample is placed in the test container (e.g. a standard UV-vis cuvette) and optically compared with colour standards, ranging in value from 0.5 to 8.0 (see Table 2).

**Table 2: ASTM D1500 colour standards.**

| ASTM Color | Chromaticity Coordinates*^{A}* (RGB USC system)*^{B}* | | | Luminous Transmittance (CIE*^{C}* Standard Source C) *T_{w}* |
|---|---|---|---|---|
| | Red | Green | Blue | |
| 0.5 | 0.462 | 0.473 | 0.065 | 0.86 ± 0.06 |
| 1.0 | 0.489 | 0.475 | 0.036 | 0.77 ± 0.06 |
| 1.5 | 0.521 | 0.464 | 0.015 | 0.67 ± 0.06 |
| 2.0 | 0.552 | 0.442 | 0.006 | 0.55 ± 0.06 |
| 2.5 | 0.582 | 0.416 | 0.002 | 0.44 ± 0.04 |
| 3.0 | 0.611 | 0.388 | 0.001 | 0.31 ± 0.04 |
| 3.5 | 0.640 | 0.359 | 0.001 | 0.22 ± 0.04 |
| 4.0 | 0.671 | 0.328 | 0.001 | 0.152 ± 0.022 |
| 4.5 | 0.703 | 0.296 | 0.000 | 0.109 ± 0.016 |
| 5.0 | 0.736 | 0.264 | 0.000 | 0.081 ± 0.012 |
| 5.5 | 0.770 | 0.230 | 0.000 | 0.058 ± 0.010 |
| 6.0 | 0.805 | 0.195 | 0.000 | 0.040 ± 0.008 |
| 6.5 | 0.841 | 0.159 | 0.000 | 0.026 ± 0.006 |
| 7.0 | 0.877 | 0.123 | 0.000 | 0.016 ± 0.004 |
| 7.5 | 0.915 | 0.085 | 0.000 | 0.0081 ± 0.0016 |
| 8.0 | 0.956 | 0.044 | 0.000 | 0.0025 ± 0.0006 |

| | | | | |
|---|---|---|---|---|
| *^{A}*Tolerances on the chromaticity coordinates are ±0.006. *^{B}*Judd, D. B., "A Maxwell Triangle Yielding Uniform Chromaticity Scales," *Journal* *of Research of the National Bureau of Standards,* Vol 14, 1935, p. 41. (RP 756): Journal of the Optical Society of America, Vol 25, 1935, p. 24. *^{C}* Commission Internationale de l'Echairage (international Commission on Illumination). | | | | |

The comparison with the standards can be made automatically by using software tools compatible with lab scale spectrophotometers. For these tests, IPT has used the Spectrophotometer ColorLite sph850, a color measuring instrument suitable for a wide range of applications. The software controlling the equipment gives the colour difference to the standards specified in ASTM D1500 (see Table 2).

For the purpose of these experiments, this method has been used for the monitoring the polymerization reaction of acetaldehyde in basic aqueous media. This reaction is a model for the study of red oil formation in acid gas scrubbers in cracker plants.

As the polymerization reaction progresses, the colour of the solution will evolve from colourless to yellow and finally to red/brown. The solutions are often turbid due to the dispersion of the polymers in the liquid phase. The final colour will depend on the red oil's molecular weight and chemical structure. If the reaction is left unattended for several weeks, an aldehyde resin (solid) material is finally formed.

### b) Sample preparation

Experiments were done preparing three batch solutions of 500 mL of NaOH at 8 wt% in distilled water. The first solution was kept as blank.

The second solution (uninhibited solution, i.e. without the addition of any inhibitor) was prepared by adding to the blank solution 1000 ppm (0.1 wt%) acetaldehyde.

The third solution (acetone inhibited solution; i.e. acetone was added as inhibitor) was prepared by adding to the blank solution 1000 ppm (0.1 wt%) acetaldehyde and the same weight equivalent acetone, 1000 ppm (0.1 wt%).

The three batch samples were allowed to stand without stirring at room temperature during the whole experiment time. Samples from each of these batches were taken once per day during at least ten days for their colour analysis according to ASTM D1500.

### c) Experimental results

The colour of each sample was evaluated according to ASTM D1500 - Standard Test Method for ASTM Color of Petroleum Products (ASTM Color Scale).

The measured results are shown in Table 3:

**Table 3: Colour scale values of solutions based on ASTM D1500**

| **Days** | **ASTM D1500 Colour Scale** | | |
|---|---|---|---|
| | **Blank solution*** | **Uninhibited solution**** | **Acetone inhibited solution***** |
| 0 | 0.25 | 1.62 | 0.99 |
| 1 | 0.47 | 3.59 | 2.23 |
| 2 | 0.71 | 4.30 | 2.62 |
| 6 | 0.8 | 5.1 | 3.1 |
| 7 | 0.5 | 4.7 | 3.2 |
| 8 | 0.88 | 4.91 | 3.76 |
| 9 | 0.95 | 4.79 | 3.58 |

| | | | |
|---|---|---|---|
| **8 wt% NaOH in distilled water; **8 wt% NaOH, 0.1 wt% acetaldehyde in distilled water; ***8 wt% NaOH, 0.1 wt% acetaldehyde, 0.1 wt% acetone in distilled water . Samples stored at RT.* | | | |

The colour test results according to ASTM D1500 are shown in the diagram of **Fehler! Verweisquelle konnte nicht gefunden werden.2.** In addition, illustrative pictures of the visual appearance of the solutions on selected days are also shown. The results show clearly that the addition of acetone effectively decreases the red oil polymer formation.

## Claims

1. Use of at least one ketone as inhibitor for aldol condensation of at least one aldehyde present in a gas scrubbing solution, in particular an acid gas scrubbing solution.

2. Use according to claim 1, **characterized in that** the at least one ketone is selected from a group of compounds of the general formulae (I)
R¹-CO-CHR²R³
wherein the moiety R¹ is selected from a group comprising linear or branched C₁-C₁₀ alkyl, C₃-C₁₀-cycloalkyl and linear or branched C₁-C₁₀-alkyl substituted C₃-C₁₀-cycloalkyl"
wherein the moieties R² and R³ are selected from a group comprising H, linear or branched C₁-C₁₀ alkyl, C₃-C₁₀-cycloalkyl and linear or branched C₁-C₁₀-alkyl substituted C₃-C₁₀-cycloalkyl, and
wherein R¹, R² and R³ can be the same or different, or
wherein R¹ and R² form together a C₄-C₁₀ cycloalkyl ring.

3. Use according to claim 2, **characterized in that** the moietiy R¹, is selected from the group comprising linear or branched C₁-C₅ alkyl and C₃-C₇-cycloalkyl, in particular ethyl, propyl, isopropyl, butyl or iso-butyl.

4. Use according to claim 2 and 3, **characterized in that**, the moieties R² and R³ are selected from a group comprising H, linear or branched C₁-C₅ alkyl and C₃-C₇-cycloalkyl, in particular H, ethyl, propyl, isopropyl, butyl or iso-butyl.

5. Use according to one of the preceding claims, **characterized in that** the at least one ketone is acetone, butan-2-one, pentan-2-one, pentan-3-one, hexan-2-one, hexan-3-one, 2-methyl-pentan-3-one, 2,2-dimethylpentan-3-one, 3-methyl-butanone, 3,3-dimethyl-butanone, cyclopentanone, cyclohexanone, 2-methyl-cyclohexanone.

6. Use according to one of the preceding claims, **characterized in that** the at least one aldehyde is at least one low molecular weight aldehyde and/or higher molecular weight aldehydes.

7. Use according to one of the preceding claims, **characterized in that** the at least one aldehyde is acetaldehyde, propanaldehyde, butanaldehyde, hexanal, heptanal, octanal nonanal and/or decanal.

8. Use according to one of the preceding claims, **characterized in that** the at least one ketone is used as inhibitor for at least one ester, preferably an ester comprising at least one double bond, in particular preferably at least one vinyl ester.

9. Use according to one of the preceding claims, **characterized in that** the at least one ketone is added to the gas scrubbing solution in stoichiometric amount in respect to the at least one aldehyde in the gas scrubbing solution.

10. Method for inhibiting aldol condensation of aldehydes, in particular low molecular weight aldehydes, in a gas scrubber solution, in particular an acid gas scrubber solution,
**characterized in that**
at least one ketone is fed to the gas scrubbing solution, in particular to the acid gas scrubbing solution.

11. Method according to claim 10, **characterized in that** the at least one ketone is a ketone as defined in one of the claims 2-5.

12. Method according to claim 10 or 11, **characterized in that** the at least one ketone is fed to the gas scrubbing solution in a molar amount that is at least substantial the same as the molar amount of the at least one aldehyde in the gas scrubbing solution.

13. Method according to one of the claims 10 to 12, **characterized in that** the at least one ketone is fed to the gas scrubbing solution in a basic media.

14. Method according to one of the claims 10 to 13, **characterized in that** the at least one ketone is fed to the gas scrubbing solution in a NaOH containing solution.

15. Method according to one of the claims 10 to 14, **characterized in that** the at least one ketone is fed at different feeding points to the gas scrubber and/or gas scrubber solution.
